(19) **Europäisches Patentamt / European Patent Office / Office européen des brevets**

(11) **EP 4 231 315 A1**

(12) # EUROPEAN PATENT APPLICATION
published in accordance with Art. 153(4) EPC

(43) Date of publication:
**23.08.2023 Bulletin 2023/34**

(21) Application number: **20957816.0**

(22) Date of filing: **23.11.2020**

(51) International Patent Classification (IPC):
**G21F 1/10** $^{(2006.01)}$    **G21F 3/02** $^{(2006.01)}$
**G21F 5/00** $^{(2006.01)}$    **C12N 1/16** $^{(2006.01)}$
**C12N 11/02** $^{(2006.01)}$    **C12R 1/645** $^{(2006.01)}$

(52) Cooperative Patent Classification (CPC):
**C12N 1/16; C12N 11/02; G21F 1/10; G21F 3/02; G21F 5/00;** C12R 2001/645

(86) International application number:
**PCT/KR2020/016629**

(87) International publication number:
**WO 2022/080572 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.10.2020 KR 20200132329**

(71) Applicant: **Coenbio Co., Ltd.**
**Seongnam-si, Gyeonggi-do 13207 (KR)**

(72) Inventors:
- **YUM, Kyu Jin**
 **Anyang-si, Gyeonggi-do 14106 (KR)**
- **LEE, Jung A**
 **Seoul 06356 (KR)**

(74) Representative: **Danubia Patent & Law Office LLC**
**Bajcsy-Zsilinszky út 16**
**1051 Budapest (HU)**

(54) **ANTI-RADIATION COMPOSITION USING MICROORGANISMS, AND ANTI-RADIATION MATERIAL COMPRISING SAME**

(57) The present invention provides a composition for radiation shield including one or more microorganisms selected from the group consisting of *Cladosporium* sp. microorganism, *Phanerochaete* sp. microorganism and *Trichosporon* sp. Microorganism, and a radiation shield material including the composition.

**FIG. 3**

(a)

**(Cont. next page)**

EP 4 231 315 A1

**FIG. 3**

(b)

## Description

### Technical Field

The present invention relates to a composition for radiation shield using microorganisms and a radiation shield material including the composition

### Background Art

Radioactive ray is generally an electromagnetic wave or particle beam generated when a radioactive material (radioactive isotope) decays into a stable material, and gamma ray (γ-ray), X-ray, neutron ray (neutron), beta-ray (β-ray), alpha-ray (α-ray) and the like fall within this category.

Radioactive ray is mainly generated in places where radioactive materials are handled, such as nuclear medicine-related hospitals, nuclear power plants, nuclear waste treatment facilities, military nuclear material facilities, and non-destructive industries. When exposed to a large amount of these radiations or exposed to the radiation for a long period of time, DNA is damaged and is fatal to the human body, so effective radiation shielding is a very important factor for workers.

The handling of radioactive materials is increasing year by year, and the global radiation shielding-related market is currently known to be in about 1,700 trillion won scale.

Radiation shield materials have long been manufactured using heavy metal powders such as lead or lead compounds. Even now, most radiation shield materials contain still a large amount of lead. However, lead dust components not only is harmful to the human body, but also causes environmental pollution due to heavy metal waste. Therefore, the use of heavy metal products such as lead is regulated mainly in the United States, Europe and the like. In addition, the shield material made of lead is not only heavy, but also uncomfortable to wear, which limits the activity of workers, and manufacturing cost is very high.

In connection with lead-free radiation shield materials, Korean Patent Registration No. 10-1145703 discloses a radiation shield sheet manufactured by mixing and curing a silicone polymer, tourmaline, barium sulfate and a silicone curing accelerator.

Korean Patent Registration No. 10-2035512 discloses a coating agent for radiation shield in which a radiation shielding agent such as tungsten, bismuth, barium, europium, dysprosium, and strontium is mixed with a binder resin such as epoxy or urethane, but there is still a problem of environmental pollution because of using heavy metals as a shielding agent, and the materials used are also expensive.

Korean Patent Registration No. 10-1731785 discloses a hydrogel matrix in which a first polymer such as alginate, chitosan, hyaluronic acid and a second polymer such as polyacrylamide, polyvinyl alcohol, and polyethylene are cross-linked in order to impart ductility and elasticity to a radiation shield material, but metal particles such as boron, lithium, gadolinium, samarium, europium, cadmium, dysprosium, lead, iron, and tungsten are used as materials for shielding radiation.

Although the above-mentioned prior arts contain a polymer material for radiation shield materials, components for shielding radiation are substantially dependent on heavy metals and may still be harmful to the human body or the environment.

### Disclosure

### Technical Problem

It is an object of the present invention to provide a composition for radiation shield and a radiation shield material that have excellent radiation shielding ability, are harmless to the human body, has light weight even without environmental pollution, is comfortable to wear, and can be manufactured at a low price.

### Solution to Problem

The present invention provides a composition for radiation shield including one or more microorganisms selected from the group consisting of *Cladosporium* sp. microorganism, *Phanerochaete* sp. microorganism and *Trichosporon* sp. microorganism.

In one embodiment, the *Cladosporium* sp. microorganism may be *Cladosporium cladosporioides,* and in one example, *Cladosporium cladosporioides* Ceb-RadF-001 strain (accession number: KCCM12440P) can be used.

In one embodiment, the *Phanerochaete* sp. microorganism may be *Phanerochaete chrysosporium* or *Phanerochaete sordida,* and in one example, *Phanerochaete chrysosporium*)Y-2 strain (accession number: KCCM10725P) can be used.

In one embodiment, the *Trichosporon* sp. microorganism may be *Trichosporon loubieri,* and in one example, *Trichosporon loubieri* Y1-A strain (accession number: KCTC10876BP) can be used.

Meanwhile, the present invention provides a radiation shield material including the composition for radiation shield.

In one embodiment, the radiation shield material may include a microorganism immobilized media.

The microorganism immobilized media may be a porous material, but is not limited thereto.

The microorganism immobilized media may be selected from polymer resin, activated carbon, zeolite, non-woven fabric or woven fabric, but is not limited thereto.

The microorganism immobilized media may further include carbon nanotubes or graphene.

**Advantageous Effects of Invention**

The microorganism of the composition for radiation shield according to the present invention not only has strong resistance to radiation, but also absorbs radiation and grows using it as growth energy, so the radiation shielding effect is very excellent, and the shielding effect is comparable to or more excellent than shield materials made of lead.

Further, since the radiation shield material according to the present invention does not use lead or other metal powder as a material, it is harmless to the human body and has no problem of environmental pollution even at the disposal. Compared to conventional shield materials, it is remarkably lighter and more flexible, and is comfortable for the activity of workers, so that it has high utility value as a material or a construction material for radiation shielding clothing, aprons, gloves, shoes and the like.

**Brief Description of Drawings**

FIG. 1 shows the arrangement of microbial samples according to the irradiation distance in the radiation (gamma ray) irradiating room carried out in the present invention.

FIG. 2 shows the change of each microbial strain with an increase in adiation (gamma ray) dose in the experimental example of the present invention.

FIG. 3 (a) is a photograph of the growth of a *Cladosporium cladosporioides* strain to which radioactive rays are not irradiated, and (b) is a photograph of the growth of a *Cladosporium cladosporioides* strain having an absorbed dose of 392 Gy.

FIG. 4 shows a porous polyurethane filter used for attaching microorganisms of the present invention.

FIG. 5 shows the arrangement of microbial samples according to the irradiation distance in the radiation (gamma ray) irradiating room carried out in the present invention.

FIG. 6 shows a radiation shielding rate measuring mechanism, in which (a) shows a radiation shielding rate measuring device, and (b) shows a radiation shielding rate measuring sensor.

**Best Mode for Carrying out the Invention**

As used herein, the term 'radiation' includes ionizing radiation, and includes gamma rays, X-rays, neutron rays, alpha rays, and beta rays.

As used herein, the term 'microorganism' is 'strain' or includes 'strain' unless otherwise stated.

As used herein, the term "single microorganism" means a microorganism composed of only one strain.

As used herein, the term "complex microorganism" means a group of microorganisms consisting of two or more kinds of strains. The complex microorganisms include those obtained by mixing a culture solution of a single microorganism or culturing two or more kinds of strains in the same medium.

As used herein, the term 'low dose', 'medium dose' and 'high dose' related to radiation are only a term used in the examples of the present invention to compare the radiation shielding effects according to the magnitude of the radiation dose with each other, and do not mean the absolute range of toxicity. The dose unit 'Gy' (Gray) is a unit representing the radiation energy absorbed by a material, and when 1 joule (J) per 1 kg of material is absorbed, it is defined as 1 Gy. For example, when exposed to 1 Gy or more of radiation at a time, acute radiation sickness appears in almost everyone. If systemic exposure doses are extremely high (20 Gy or more), severe acute neurovascular disease can occur. For reference, the highest radiation exposure dose of power plant workers in the Chernobyl accident was 16 Gy. In the examples of the present invention, 220.6 Gy and 392 Gy are expressed as low doses, but this is relatively low compared to 21,687 Gr and 38,600 Gy, and is only expressed, which is equivalent to tens to hundreds of times the lethal dose.

The present inventors have studied various microorganisms in order to select microorganisms capable of effectively blocking high-risk radiation such as gamma rays and X-rays, and as a result, confirmed that the radiation shielding ability was remarkably excellent in three types of microorganisms.

The present invention provides a composition for radiation shield including one or more microorganisms selected from the group consisting of *Cladosporium* sp. microorganism, *Phanerochaete* sp. microorganism and *Trichosporon* sp. microorganism.

The composition for radiation shield of the present invention may include the above strains alone (single microorganism), or may include as a mixture of the above strains (complex microorganism).

The composition for radiation shield may be the strain itself, or a culture solution or a mixed solution thereof, and the strain, culture solution, and mixed solution may be in a semi-dried or dried form as necessary.

The *Cladosporium* sp. Microorganism is known as black fungi that produce melanin pigments, and *Cladosporium cladosporioides* is preferable, but is not limited thereto. In one example, it may be a *Cladosporium cladosporioides* Ceb-RadF-001 strain. The *Cladosporium cladosporioides* Ceb-RadF-001 strain was deposited at the Korean Culture Center of Microorganisms under the accession number KCCM12440P on February 26, 2019.

The *Phanerochaete* sp. Microorganism is known as white fungi, and *Phanerochaete chrysosporium* or *Phanerochaete sordida* is preferable, but is not limited thereto. In one example, *Phanerochaete* sp. Y-2 strain can be used. The *Phanerochaete* sp. Y-2 strain was deposited at the Korean Culture Center of Microorganisms under the accession number KCCM10725P on February 26, 2019.

The *Trichosporon* sp. microorganism is yeast, and *Trichosporon loubieri* can be preferably used, but is not limited thereto. In one example, *Trichosporon loubieri* Y1-A strain can be used. The *Trichosporon loubieri* Y1-A strain was deposited at the Korean Culture Center of Microorganisms under the accession number KCCM10876BP on February 26, 2019.

The microorganisms according to the present invention can be cultured in a medium. Natural medium or synthetic medium may be used as the culture medium. The culture medium may include a carbon source, a nitrogen source, and inorganic salts.

The carbon source of the culture medium is not limited, but can be carbon sources known in the field of microbial culture, for example, sugars such as glucose, sucrose, fructose, maltose, lactose, dextrin, dextrose, starch, organic acids such as malic acid and citric acid, fatty acids having a low molecular weight, and the like.

The nitrogen source of the culture medium is not limited, but can be nitrogen sources known in the field of microbial culture, for example, peptone, meat extract, yeast extract, dried yeast, casein, whey protein, soybean, ammonium salt, nitrate and other organic or inorganic nitrogen, sulfur-containing compounds and the like.

The inorganic salt of the culture medium is not limited, but can be inorganic salts known in the field of microbial culture, for example, magnesium (Mg) manganese (Mn), calcium (Ca) iron (Fe), potassium (K), sodium (Na), phosphorus (P), sulfur (S), boron (B), molybdenum (Mo). , copper (Cu), cobalt (Co), zinc (Zn) and the like,

The culture medium may further contain growth factors, if necessary, in addition to components of a carbon source, a nitrogen source and an inorganic salt. The growth factor may be amino acid, vitamin, nucleic acid, or compounds related thereto.

The microorganisms according to the present invention are not limited, but may be cultured in a temperature range of 20°C to 40°C, preferably in a culture temperature range of 25°C to 35°C.

The microorganism according to the present invention is not limited, but may be cultured for 12 hours to 7 days, preferably for 12 hours to 5 days.

The composition for radiation shield of the present invention may include a single microorganism or a complex microorganism. The complex microorganism of the present invention may be prepared by mixing a culture solution obtained by culturing each strain individually, or may be prepared by culturing two or more strains together.

The concentration of microorganisms in the composition for radiation shield of the present invention is not limited, but the concentration may be $0.5 \times 10^2$ CFU/ml or more, preferably $0.5 \times 10^3$ CFU/ml or more, more preferably $0.5 \times 10^4$ CFU/ml or more, and still more preferably $0.5 \times 10^5$ CFU/ml or more.

In one embodiment, the composition for radiation shield of the present invention may be a culture medium of microorganisms or a microorganism concentrated or purified from the culture medium, and may be in a dry or semi-dried form, if necessary.

The composition for radiation shield of the present invention may contain an acceptable carrier, if necessary. The acceptable carriers are those suitable as a nutrient for the selected microorganisms. For example, physiological saline, sterile water, buffered saline, dextrose solution, maltodextrin solution, glycerol, and one or more of these components may be mixed and used. Other conventional additives such as antioxidants, buffers, and bacteriostatic agents may be added as needed. In addition, a diluent, a dispersant, a surfactant, a binder, and a lubricant may be additionally added to form a liquid formulation such as an aqueous solution, a suspension, an emulsion, or a solid formulation such as a powder.

Meanwhile, the present invention provides a radiation shield material including the composition for radiation shield. The radiation shield material of the present invention may include a microorganism immobilized media. The microbial immobilized media can be used for the purpose of maintaining the shape of a shield material while allowing microorganism to attach well. In one embodiment, the microorganism-immobilized media may be a porous material such as a polymer resin in a matrix form having pores, activated carbon, zeolite, nonwoven fabric, woven fabric, and the like. The polymer resin is not limited, but may be, for example, synthetic polymers such as polyurethane, polyacrylamide, polyethylene glycol, and polyvinyl alcohol, or natural polymers such as agar, agarose, k-carrageenan, alginate, and chitosan. The shape of the microbial immobilized media is not limited, but may be foam, sponge, filter, sheet, or film, and if necessary, it may be in powder or pellet form. In one embodiment, the composition for radiation shield may be supported on a microorganism-immobilized media to stabilize attachment, and if necessary, additional cultivation can be performed. In addition, it may be semi-dried or dried, if necessary.

In one embodiment, the radiation shield material according to the present invention may be prepared by impregnating a microorganism-immobilized media in the composition for radiation shield (microbial culture solution or concentrate). The impregnation time is to ensure that microorganisms are sufficiently attached to the media, and it can be appropriately adjusted according to the type of microorganism and media. The impregnation time may be 12 hours or more, but is not limited thereto. The impregnation time may be preferably for 1 to 15 days. If necessary, a semi-drying or drying process may be added after impregnation. Drying is preferably natural drying.

In one embodiment, the microorganism immobilized media may be prepared by a known entrapment method or encapsulation method.

The microorganism immobilized media may further include carbon nanotubes or graphene that is known to have a radiation shielding effect.

The composition for radiation shield or a radiation shield material according to the present invention can be used in various ways as materials such as clothing for radiation shielding (shield clothing), shield aprons, shield gloves, shield hats, shield shoes, shield sheets, and shield panels. At this time, the composition for radiation shield or a radiation shield material may be used as an inner material of a shielding product.

Hereinafter, the present invention will be described in more detail with reference to examples. However, these examples are for illustrative purposes only, and the scope of the present invention is not limited thereto.

**Example 1: Production of composition for radiation shield containing single microorganism (Phanerochaete chrysosporium strain)**

*Phanerochaete chrysosporium* Y-2 strain (accession number: KCCM10725P) was cultured in a BBM medium (Bold's basal medium to which $KH_2PO_4$ 0.175g, $CaCl_2.2H_2O$ 0.025g, $MgSO_4.7H_2O$ 0.075g, $NaNO_3$ 0.25g, $K_2HPO_4$ 0.075g, NaCl 0.025g, $Na_2EDTA$ 0.1g, KOH 0.062g, $FeSO_4.7H_2O$ 0.0498g, $H_3BO_3$ 0.115g, $MnCl_2.4H_2O$ 0.00181g, $ZnSO_4.7H_2O$ 0.000222g, $NaMoO_4.5H_2O$ 0.00039g, $CuSO_4.5H_2O$ 0.000079g, $Co(NO_3)_2.6H_2O$ 0.0000494g were respectively added to 1 liter of purified water.

**Example 2: Production of composition for radiation shield containing single microorganism (Trichosporon loubieri strain)**

*Trichosporon loubieri* Y1-A strain (accession No.: KCTC10876BP) was cultured in a PDB medium to which 200 g of potato infusion and 20 g of dextrose was respectively added to 1 liter of purified water, by using a shaking incubator at 130 rpm and 30°C for 20 hours. Culturing was stopped when the microbial growth curve was in the middle of the logarithmic phase and the normal phase, and was used for subsequent experiments.

**Example 3: Production of composition for radiation shield containing single microorganism (Cladosporium cladosporioides strain)**

*Cladosporium cladosporioides* Ceb-RadF-001 strain (accession No.: KCCM12440P) was cultured in a NB(nutrient broth) medium to which 3g of beef extract, 5g of peptone, and 8g of NaCl were added to 1 liter of purified water, by using a shaking incubator at 130 rpm and 30°C for 20 hours. Culturing was stopped when the microbial growth curve was in the middle of the logarithmic phase and the normal phase, and was used for subsequent experiments.

**Example 4: Production of composition for radiation shield containing two kinds of microorganisms**

*Phanerochaete chrysosporium* Y-2 strain culture solution produced in Example 1 and *Trichosporon loubieri* Y1-A strain culture solution produced in Example 2 were mixed in a ratio of 1:1 to prepare a two-kind complex microorganism culture solution.

**Example 5: Production of composition for radiation shield containing two kinds of microorganisms**

*Phanerochaete chrysosporium* Y-2 strain culture solution produced in Example 1 and *Cladosporium cladosporioides* Ceb-RadF-001 strain culture solution produced in Example 3 were mixed in a ratio of 1:1 to prepare a two-kind complex microorganism culture solution.

**Example 6: Production of composition for radiation shield containing two kinds of microorganisms**

*Trichosporon loubieri* Y1-A strain culture solution produced in Example 2 and *Cladosporium cladosporioides* Ceb-RadF-001 strain culture solution produced in Example 3 were mixed in a ratio of 1:1 to prepare a two-kind complex microorganism culture solution.

**Example 7: Production of composition for radiation shield containing three kinds of microorganisms**

*Phanerochaete chrysosporium* Y-2 strain culture solution produced in Example 1, *Trichosporon loubieri* Y1-A strain culture solution produced in Example 2 and *Cladosporium cladosporioides* Ceb-RadF-001 strain culture solution produced in Example 3 were mixed in a ratio of 1:1:1 to prepare a three-kind complex microorganism culture solution.

**Experimental Example 1: Measurement of the survival rate of microbial strains according to radiation irradiation**

(1) Preparation of sample

200 mL of each of the microorganism-containing compositions prepared in Examples 1 to 3 was added to a 0.5 L Erlenmeyer flask having a baffle, and microbial samples were prepared by closing with a hydrophobic silicon stopper having air permeability and a small amount of water evaporation. Three microbial samples (3 repetitions) were made by the kinds of microorganims.

(2) Radiation irradiation

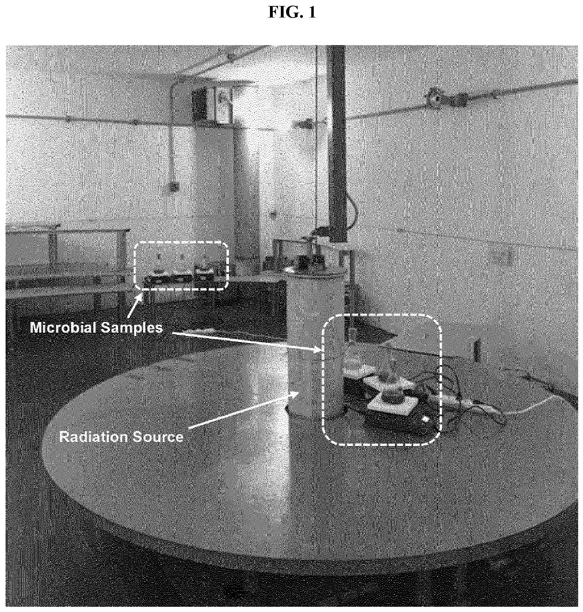

As shown in FIG. 1, the microbial samples were located at a distance of 25 cm and 379 cm, respectively, from a radiation source in a cobalt 60 ($^{60}$Co) radiating room, placed on a shake (DAIHAN Scentific model SHO-2D) and irradiated with radioactive ray while continuously shaking at a speed of about 100 RPM. The control was not irradiated.
The temperature of the laboratory during the experiment was maintained in the range of 21°C to 25°C without any artificial control. Mainly, the fluorescent lamp in the laboratory was turned on during business hours, and remained turned off after work ending time.
The absorbed dose rate of the sample was calculated from the radiation source of $^{60}$Co and the irradiation distance, and radioactive ray was irradiated for about 5 days for 93.08 hr.
The $^{60}$Co radiation dose conditions are shown in Table 1 below.

Table 1

| Absorbed dose rate and total absorbed dose | | | |
|---|---|---|---|
| Absorbed dose rate | Irradiation distance | Irradiation time | Absorbed dose (D) |
| 233 Gy/hr | 25 cm | 93.08 hr | $233 \text{ Gy/hr} \times 93.08 \text{ hr} = 21{,}687 \text{ Gy}$ |
| 2.37 Gy/hr | 379 cm | 93.08 hr | $2.37 \text{ Gy/hr} \times 93.08 \text{ hr} = 220.6 \text{ Gy}$ |

(3) Measuring the survival rate of microorganisms after irradiation

For *Trichosporon loubieri* strain, the viable cell count (bacterial concentration) of the microorganism was measured before irradiation (0 days) and after irradiation for about 5 days, respectively. For *Cladosporium cladosporioides* strain and *Phanerochaete chrysosporium* strain, the amount of microbial biomass was measured before irradiation (0 days) and after irradiation for about 5 days. The results are shown in Tables 2 to 4 below.
The survival rate of microorganisms was calculated as a percentage by using the strain cultured for about 5 days without irradiation (0 Gy) as a control and calculating the viable cell count (bacterial concentration) or the biomass amount of strain according to the radiation dose compared to the control.

Table 2

| Survival rate of *Cladosporium cladosporioides* strain according to Co-60 radiation dose | | | |
| --- | --- | --- | --- |
| Dose | Dry biomass (mg/100mL) | | Survival rate (%) compared to control on the day 5 |
| | 0 day | 5 day | |
| 0 Gy (Control) | 205 | 495 | 100% |
| 220.6 Gy | 205 | 531 | 107.3% |
| 21687 Gy | 205 | 289 | 58.4% |

Table 3

| Survival rate of *Phanerochaete chrysosporium* strain according to Co-60 radiation dose | | | |
| --- | --- | --- | --- |
| Dose | Dry biomass (mg/100mL) | | Survival rate (%) compared to control on the day 5 |
| | 0 day | 5 day | |
| 0 Gy (Control) | 288 | 623 | 100% |
| 220.6 Gy | 288 | 588 | 94.4% |
| 21687 Gy | 288 | 290 | 46.5% |

Table 4

| Survival rate of *Trichosporon loubieri* strain according to Co-60 radiation dose | | | |
| --- | --- | --- | --- |
| Dose | Viable cell count (CFU/mL) | | Survival rate (%) compared to control on the day 5 |
| | 0 day | 5 day | |
| 0 Gy (Control) | $1.0 \times 10^6$ | $7.1 \times 10^6$ | 100% |
| 220.6 Gy | $1.0 \times 10^6$ | $6.9 \times 10^6$ | 97.2% |
| 21687 Gy | $1.0 \times 10^6$ | $5.6 \times 10^4$ | 0.8% |

As shown in Tables 2 to 4, *Phanerochaete chrysosporium* strain and *Trichosporon loubieri* strain showed a high survival rate of more than 94% at 220.6 Gy, and *Cladosporium cladosporioides* strain showed a growth of 7.3% or more compared to the control. This is judged that the *Cladosporium cladosporioides* strain showed higher growth rate by using high energy of radiation as a growth energy source. *Phanerochaete chrysosporium* strain and *Cladosporium cladosporioides* strain survived 46.5% and 58.4%, respectively, even after absorbing high doses (21,687 Gy). For reference, it can be seen that *Deinococcus radiodurans* strain, which the US researchers reported as being very resistant to radioactivity, showed a 1% survival rate at 9,000 Gy, whereas the microorganisms of the present invention have remarkably higher viability against radiation.

**Experimental Example 2: Morphological change experiment of microorganisms according to radiation dose**

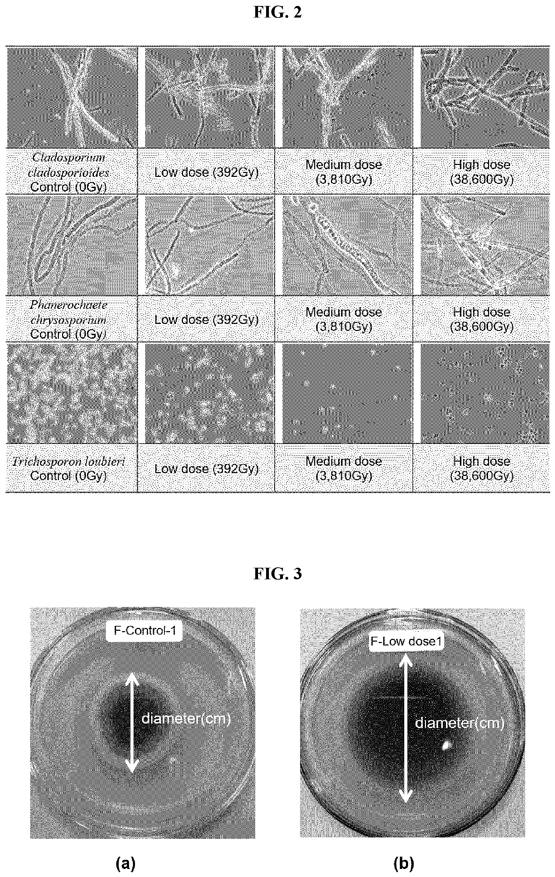

The experiment was carried out in the same manner as in Experimental Example 1, but the irradiation was carried out by increasing the dose to 392 Gy, 3,810 Gy, and 38,600 Gy, respectively, for 7 days. In order to confirm the morphological change of microbial strains according to the irradiation dose, samples collected by 10 ml were diluted $10^4$ times with sterile physiological saline, and microbial strains that survived and grown after being placed on solid medium (NA, TSA, PDA) containing nutrients for each strain were observed and shown in FIG. 2.
As shown in FIG. 2, it can be confirmed that the higher the dose, the larger the thickness and size of the mycelium and spore, and endoplasmic reticulum and nuclear (DNA) substances increased in the mycelium and spores.

**Experimental Example 3: Measurement of the growth rate of *Cladosporium cladosporioides* strain according to the radiation dose**

For the *Cladosporium cladosporioides* strain having an increased growth rate even at 220.6 Gy, the bacterial growth rate was measured again while irradiating gamma rays at 392 Gy, 3810 Gy, and 38600 Gy, respectively, for 7 days, and the results are shown in Table 5 and FIG. 3 below. Control is a strain that has not been irradiated with radiation.

Table 5

| Measurement result of bacterial growth rate after irradiation with Co-60 for 7 days | | |
|---|---|---|
| **Irradiation days** | **0 day** | **7 day** |
| **Control** | Diameter 3.60 cm | Diameter 3.90 cm |
| **Irradiated at 392 Gy** | Diameter 3.59 cm | **Diameter 5.20 cm(1.78 times increase in bacterial growth rate compared to control)** |
| **Irradiated at 3,810 Gy** | Diameter 3.61 cm | Diameter 3.10 cm |
| **Irradiated at 38,600 Gy** | Diameter 3.62 cm | Diameter 3.05 cm |

As shown in FIG. 3 and Table 5, it can be confirmed that *Cladosporium cladosporioides* strain has a growth diameter of 5.20 cm at a dose of 392 Gy, which is 1.78 times higher than that of the control. It can also be seen that it does not die even at a high dose of 38,600 Gy.

**Example 8: Preparation of radiation shield material**

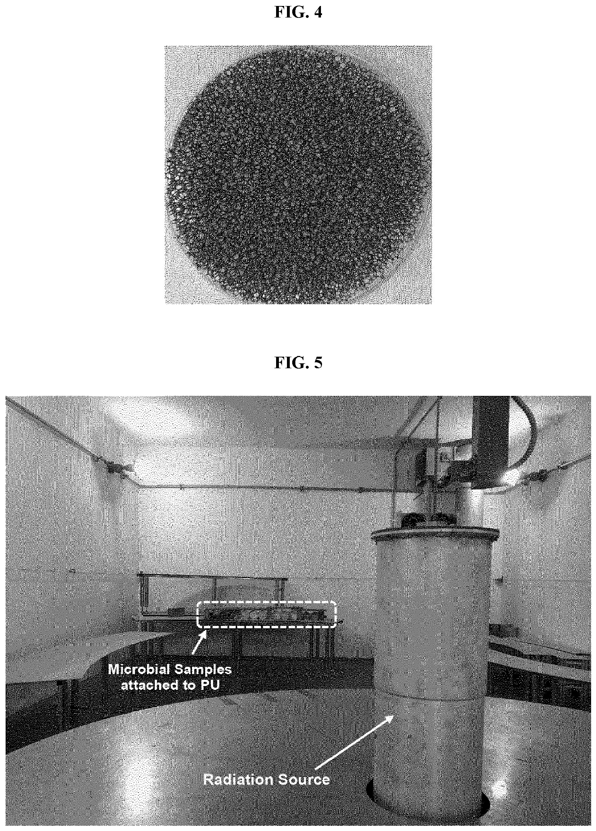

As shown in FIG. 4, the microorganism-containing composition for radiation shield prepared in Examples 1 to 7 was supported on a porous polyurethane filter having a void of 25 ppi and a thickness of 10 mm, respectively, and then dried to produce a radiation shield material sample to which each microorganism was attached.

**Experimental Example 4: Experiment of radiation shielding effect**

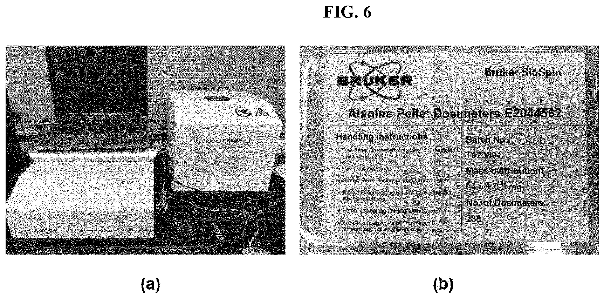

In order to measure the radiation shielding effect of the microbial strains according to the present invention, the radiation shield material sample prepared in Example 8 was placed in a $^{60}$Co radiation irradiating room as shown in FIG. 5, and irradiated with radioactive rays under the irradiation conditions for shielding ability tests shown in Table 6 below. The shielding rate measurement results are shown in Table 7 below. At this time, the radiation shielding rate measurement sensor was attached to the back of the polyurethane shielding sample using Alanine Pellet Dosimeters (Bruker BioSpin, USA), and the measurement equipment was e-scan (Bruker BioSpin, USA) (FIG. 6).

Table 6

| Irradiation condition for experimentation of radiation shielding ability | | |
|---|---|---|
| **Radiation irradiation** | Co-60 | Temperature: 22.7±1°C (irradiation room) |
| **Absorbed dose rate (Gy/hr)** | 2.3 | Light illuminance : 30-70 Lx |
| **Total irradiation time** | 48 hr | |
| **Total absorbed dose rate: 110.4 Gy** | | |

Table 7

| Measurement result of radiation shielding rate | | | | | |
|---|---|---|---|---|---|
| | **Treated group (3 repetitions)** | | **Radiation [( Shielding D0-D)/D0)]×100 Rate (%)** | | |
| **Example** | Strain | | 22hr | 48hr | **Shielding rate average(%)** |
| **Control** | - | 1 repetition | 7.714206 | 10.222980 | |
| | | 2 repetition | 5.359606 | 7.970050 | |
| | | 3 repetition | 7.847337 | 9.021764 | |
| | | Average value | **6.973717** | **9.071598** | **8.2** |
| **Example 1** | P. chrysosporium | 1 repetition | 15.006447 | 14.156591 | |
| | | 2 repetition | 14.716102 | 14.255646 | |
| | | 3 repetition | 14.467462 | 14.714976 | |
| | | Average value | **14.730004** | **14.375738** | **14.6** |
| **Example 2** | T. loubieri | 1 repetition | 15.251054 | 15.172152 | |
| | | 2 repetition | 12.580364 | 10.388004 | |
| | | 3 repetition | 12.132538 | 13.717625 | |
| | | Average value | **13.321318** | **13.092594** | **13.2** |
| **Example 3** | C. cladosporioides | 1 repetition | 28.317547 | 25.219327 | |
| | | 2 repetition | 21.776832 | 22.316921 | |
| | | 3 repetition | 22.431189 | 19.726522 | |
| | | Average value | **24.175189** | **22.420923** | **23.3** |
| **Example 4** | P. chrysosporium + T. loubieri | 1 repetition | 17.034614 | 14.978545 | |
| | | 2 repetition | 15.698547 | 15.125686 | |
| | | 3 repetition | 15.495756 | 15.354685 | |
| | | Average value | **16.076306** | **15.152972** | **15.6** |

(continued)

| Measurement result of radiation shielding rate | | | | | |
| --- | --- | --- | --- | --- | --- |
| | Treated group (3 repetitions) | | Radiation [( Shielding D0-D)/D0)]×100 Rate (%) | | |
| Example | Strain | | 22hr | 48hr | Shielding rate average(%) |
| Example 5 | P. chrysosporium + C. cladosporioides | 1 repetition | 32.546856 | 25.969869 | |
| | | 2 repetition | 25.456256 | 24.987895 | |
| | | 3 repetition | 24.966856 | 23.546686 | |
| | | Average value | **27.656656** | **24.834817** | **26.2** |
| Example 6 | T. loubieri + C. cladosporioides | 1 repetition | 30.569624 | 26.594856 | |
| | | 2 repetition | 27.695614 | 24.496256 | |
| | | 3 repetition | 25.266562 | 25.765922 | |
| | | Average value | **27.843933** | **25.619011** | **26.7** |
| Example 7 | P. chrysosporium + T. loubieri + C. cladosporioides | 1 repetition | 30.469856 | 26.995586 | |
| | | 2 repetition | 27.456253 | 25.262575 | |
| | | 3 repetition | 27.685794 | 26.096846 | |
| | | Average value | **28.537301** | **26.118336** | **27.3** |
| | Thickness 2 mm lead | | 13.668271 | 11.646847 | 12.7 |
| | Thickness 4 mm lead | | 17.361586 | 19.719506 | 18.5 |
| | Thickness 6 mm lead | | 29.168030 | 26.541714 | 27.9 |

As shown in Table 7 above, treated groups (Examples 1 to 3) to which the single microorganisms according to the present invention were attached showed a higher radiation shielding rate than that of the control and the 2 mm thick lead. In particular, the treated group to which *Cladosporium cladosporioides* strain was attached showed the highest radiation shielding rate of 23.3%, which showed a higher shielding rate than that of 4 mm thick lead. In addition, the treated group to which the complex microorganism according to the present invention was attached showed a significantly higher radiation shielding rate compared to the single microorganism, and the compositions of Examples 5 to 7 showed a radiation shielding rate comparable to that of 6 mm thick lead.

**Industrial Applicability**

The present invention relates to a composition for radiation shield using microorganisms and a radiation shield material comprising the composition.

| 0-1 | **Form PCT/RO/134** **Indications Relating to Deposited Microorganism(s) or Other Biological Material (PCT Rule 13bis)** | |
| --- | --- | --- |

(continued)

| 0-1-1 | Prepared Using | **ePCT-Filing**<br>**Version 4.7.111 MT/FOP**<br>**20201118/0.20.5.24** |
|---|---|---|
| **0-2** | **International Application No.** | PCT/KR2020/016629 |
| 0-3 | Applicant's or agent's file reference | SOP-20012-CO |

| **1** | **The indications made below relate to the deposided microorganism(s) or other biological material referred to in the description on: Paragraph number** | |
|---|---|---|
| **1-1** | | **37** |
| **1-3** | **Identification of deposit** | |
| 1-3-1 | Name of depositary institution | **KCCM Korean Culture Center of Microorganisms** |
| 1-3-2 | Address of depositary institution | **Korean Culture Center of Microorganisms (KCCM)**<br>**361-221, Yurim B/D**<br>**Honje 1**<br>**Sudaemun**<br>**Seoul 120-091**<br>**Republic of Korea** |
| 1-3-3 | Date of deposit | **26 February 2019 (26.02.2019)** |
| 1-3-4 | Accession Number | **KCCM 12440P** |
| **1-4** | **Additional Indications** | |
| **1-5** | **Designated States for Which Indications are Made** | **All designations** |
| **1-6** | **Separate Furnishing of Indications**<br>These indications will be submitted to the International Bureau later | |
| **2** | **The indications made below relate to the deposided microorganism(s) or other biological material referred to in the description on:** | |
| **2-1** | **Paragraph number** | **38** |
| **2-3** | **Identification of deposit** | |
| 2-3-1 | Name of depositary institution | **KCCM Korean Culture Center of Microorganisms** |
| 2-3-2 | Address of depositary institution | **Korean Culture Center of Microorganisms (KCCM) 361-221, Yurim B/D**<br>**Honje 1**<br>**Sudaemun**<br>**Seoul 120-091**<br>**Republic of Korea** |
| 2-3-3 | Date of deposit | **19 December 2005 (19.12.2005)** |
| 2-3-4 | Accession Number | **KCCM 10725P** |
| **2-4** | **Additional Indications** | |
| **2-5** | **Designated States for Which Indications are Made** | **All designations** |
| **2-6** | **Separate Furnishing of Indications**<br>These indications will be submitted to the International Bureau later | |

(continued)

| 3 | The indications made below relate to the deposited microorganism(s) or other biological material referred to in the description on: | |
|---|---|---|
| 3-1 | Paragraph number | 39 |
| 3-3 | **Identification of deposit** | |
| 3-3-1 | Name of depositary institution | **KCTC Korean Collection for Type Cultures** |
| 3-3-2 | Address of depositary institution | **Korean Collection for Type Cultures (KCTC)** **181 Ipsin-gil Jeongeup-si Jeollabuk-do 56212** **Republic of Korea** |
| 3-3-3 | Date of deposit | **26 February 2001 (26.02.2001)** |
| 3-3-4 | Accession Number | **KCTC 10876BP** |
| **3-4** | **Additional Indications** | |
| **3-5** | **Designated States for Which Indications are Made** | **All designations** |
| **3-6** | Separate Furnishing of Indications These indications will be submitted to the International Bureau later | |

**FOR RECEIVING OFFICE USE ONLY**

| 0-4 | **This form was received with the international application:** **(yes or no)** | |
|---|---|---|
| 0-4-1 | Authorized officer | |

**FOR INTERNATIONAL BUREAU USE ONLY**

| 0-5 | **This form was received by the international Bureau on:** | |
|---|---|---|
| 0-5-1 | Authorized officer | |

**Claims**

1. A composition for radiation shield comprising one or more microorganisms selected from the group consisting of *Cladosporium* sp. microorganism, *Phanerochaete* sp. microorganism and *Trichosporon* sp. microorganism.

2. The composition for radiation shield according to claim 1, wherein the *Cladosporium* sp. microorganism is *Cladosporium cladosporioides*.

3. The composition for radiation shield according to claim 1, wherein the *Phanerochaete* sp. microorganism is *Phanerochaete chrysosporium* or *Phanerochaete sordida.*

4. The composition for radiation shield according to claim 1, wherein the *Trichosporon* sp. microorganism is *Trichosporon loubieri.*

5. A radiation shield material comprising the composition for radiation shield according to claim 1.

6. The radiation shield material according to claim 5, wherein the radiation shield material includes a microorganism immobilized media.

7. The radiation shield material according to claim 6, wherein the microorganism immobilized media is a porous material.

8. The radiation shield material according to claim 6, wherein the microorganism immobilized media is selected from polymer resin, activated carbon, zeolite, non-woven fabric or woven fabric.

9. The radiation shield material according to claim 5, wherein the radiation shield material further includes carbon nanotubes or graphene.

**FIG. 1**

## FIG. 2

## FIG. 3

(a)                                           (b)

FIG. 4

FIG. 5

FIG. 6

(a)

(b)

## INTERNATIONAL SEARCH REPORT

| | |
|---|---|
| International application No. | |
| | **PCT/KR2020/016629** |

**A. CLASSIFICATION OF SUBJECT MATTER**

**G21F 1/10**(2006.01)i; **G21F 3/02**(2006.01)i; **G21F 5/00**(2006.01)i; **C12N 1/16**(2006.01)i; **C12N 11/02**(2006.01)i; C12R 1/645(2006.01)n

According to International Patent Classification (IPC) or to both national classification and IPC

**B. FIELDS SEARCHED**

Minimum documentation searched (classification system followed by classification symbols)

G21F 1/10(2006.01); C02F 1/44(2006.01); C12N 1/00(2006.01); G21F 9/00(2006.01); G21F 9/12(2006.01); G21F 9/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 방사선(radioactive ray), 차폐(shield), 클라도스포리움(cladosporium), 패네로키테 (phanerochaete), 트라이코스포론(trichosporon)

**C. DOCUMENTS CONSIDERED TO BE RELEVANT**

| Category* | Citation of document, with indication, where appropriate, of the relevant passages | Relevant to claim No. |
|---|---|---|
| Y | SHUNK, Graham K. et al. A Self-Replicating Radiation-Shield for Human Deep-Space Exploration: Radiotrophic Fungi can Attenuate Ionizing Radiation aboard the International Space Station. In: bioRxiv. 1 7 July 2020, pages 1-16. <doi: https://doi.org/10.1101/2020.07.16.205534.>. See abstract, and page 3. | 1-9 |
| Y | KR 10-2020-0005048 A (COENBIO CO., LTD.) 15 January 2020 (2020-01-15) See paragraphs [0019]-[0020], and claims 2 and 9. | 1-9 |
| Y | KR 10-2017-0123044 A (SOGANG UNIVERSITY RESEARCH & BUSINESS DEVELOPMENT FOUNDATION) 07 November 2017 (2017-11-07) See abstract, claim 6, and figure 1. | 6-8 |
| A | KR 10-2015-0003619 A (SOGANG UNIVERSITY RESEARCH & BUSINESS DEVELOPMENT FOUNDATION) 09 January 2015 (2015-01-09) See entire document. | 1-9 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 July 2021** | **12 July 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2020/016629** |

**C.     DOCUMENTS CONSIDERED TO BE RELEVANT**

| Category* | Citation of document, with indication, where appropriate, of the relevant passages | Relevant to claim No. |
|---|---|---|
| A | JP 2013-174557 A (BIO TECHNO NET CO., LTD. et al.) 05 September 2013 (2013-09-05)<br>    See entire document. | 1-9 |

Form PCT/ISA/210 (second sheet) (July 2019)

**INTERNATIONAL SEARCH REPORT**
Information on patent family members

International application No.

**PCT/KR2020/016629**

| Patent document cited in search report | | | Publication date (day/month/year) | Patent family member(s) | | | Publication date (day/month/year) |
|---|---|---|---|---|---|---|---|
| KR | 10-2020-0005048 | A | 15 January 2020 | KR | 10-2213178 | B1 | 05 February 2021 |
| KR | 10-2017-0123044 | A | 07 November 2017 | | None | | |
| KR | 10-2015-0003619 | A | 09 January 2015 | KR | 10-1598607 | B1 | 29 February 2016 |
| | | | | US | 2015-0004674 | A1 | 01 January 2015 |
| JP | 2013-174557 | A | 05 September 2013 | | None | | |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101145703
- KR 102035512
- KR 101731785